# EUROPEAN PATENT APPLICATION

(11) **EP 2 151 172 A1**
(43) Date of publication of application: **10.02.2010**
(21) Application number: 08764684.0
(22) Date of filing: 26.05.2008
(51) Int. Cl.: A45D 26/00, A61N 5/06

(54) **LIGHT DEPILATION DEVICE**

(30) Priority: 28.05.2007 JP 2007141127
(71) Applicant: Panasonic Electric Works Co., Ltd, Kadoma-shi Osaka 571-8686 (JP)
(72) Inventor: FUJIKAWA, Shoji, Kadoma-shi, Osaka (JP)
(74) Representative: Müller-Boré & Partner Patentanwälte
(86) International application number: PCT/JP2008/059651
(87) International publication number: WO 2008/146786

(57) **Abstract**

An optical depilation apparatus is used for performing depilation by irradiating skin with light. The apparatus has: a light source unit 20; a light irradiation port 3; and an apparatus body 1 emitting light from the light source unit 20 through the light irradiation port 3; a light shielding part 6 disposed so as to surround the light irradiation port 3 of the apparatus body 1; and a support part that supports the light shielding part 6 so as to allow the light shielding part to move in the light irradiation direction with respect to the apparatus body 1. The support part has a receiving portion 9 provided at the apparatus body 1, a convex portion 7 provided at the light shielding part 6, and a spring member 10 disposed between the receiving part 9 and the convex part 7. Light leak is prevented whereby safety is improved.

## Description

### Technical Field

The present invention relates to an optical depilation apparatus that performs depilation by irradiating skin with light.

### Background Art

An optical depilation apparatus in which hair papilla is heated by transferring light energy emitted by a laser diode or a flashlight to the hair, thereby making it difficult for nutrients to reach the hair, is known as a device for performing depilation. In such an optical depilation apparatus, irradiation with light has to be conducted from the direction perpendicular to the skin surface. Accordingly, in the optical depilation apparatus disclosed in Patent Document 1 or Patent Document 2 below, a plurality of contact sensors are disposed in positions surrounding the laser beam irradiation portion of the distal end of the apparatus, the optical depilation apparatus is determined to have a perpendicular posture when the contact sensors detect contact with the skin surface, and the laser beam irradiation is then started.

However, with the above-described configuration, although the posture of the optical depilation apparatus during light irradiation can be accurately corrected, safety measures taken to prevent the light from leaking to the outside are insufficient. In particular, in a case where a flashlight, which generates flashes, is used as the light source, the flashes can leak to the outside and enter the eyes. Therefore, more reliable safety measures are needed.
Patent Document 1: Japanese Unexamined Patent Publication No. 2005-278724.
Patent Document 2: Japanese Unexamined Patent Publication No. 2006-525036.

### Disclosure of the Invention

It is an object of the present invention to provide an optical depilation apparatus that resolves the above-described problems.

It is another object of the present invention to provide an optical depilation apparatus that can reliably prevent light leak and can perform depilation with high safety.

An optical depilation apparatus according to one aspect of the present invention is an optical depilation apparatus for performing depilation by irradiating skin with light, including: an apparatus body having a light source unit and a light irradiation port and emitting light from the light source unit through the light irradiation port; a light shielding part disposed so as to surround the light irradiation port of the apparatus body; and a support part that supports the light shielding part so as to allow the light shielding part to move in a light irradiation direction with respect to the apparatus body.

### Brief Description of the Drawings

Figs. 1A and 1B are cross-sectional views illustrating schematically the optical depilation apparatus of one embodiment of the invention. Fig. 1A shows a state in which the light shielding part is not pressed against the skin. Fig. 1B shows a state in which the light shielding part is pressed against the skin.
Figs. 2A to 2C illustrate the external appearance of the optical depilation apparatus. Fig. 2A is a front view, Fig. 2B is a side view, and Fig. 2C is a plan view.
Figs. 3A and 3B show schematically the optical depilation apparatus in a state in which the light shielding part is pushed down. Fig. 3A is a front view. Fig. 3B is a cross-sectional view in a state in which the apparatus is viewed from one side.
Figs. 4A and 4B are explanatory drawings of a switch structure using a switch spring in the optical depilation apparatus.

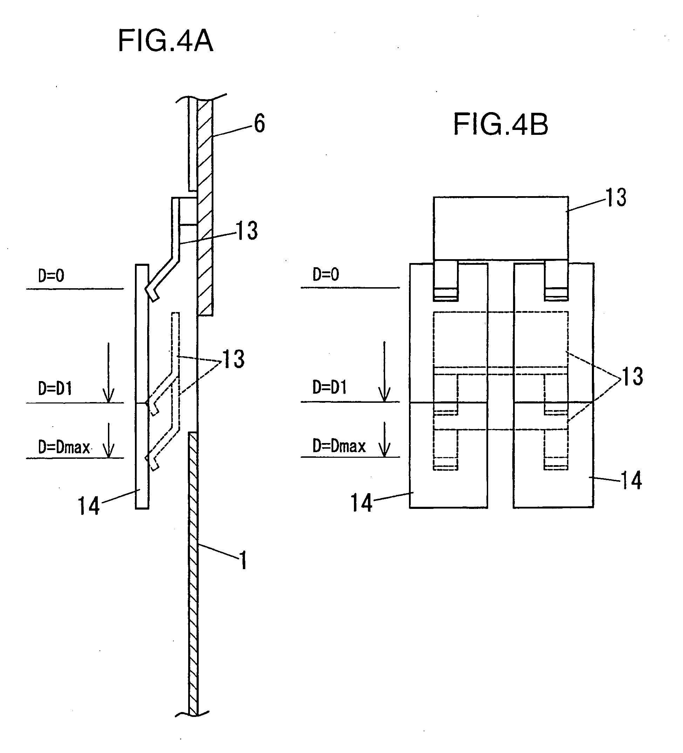

Fig. 4A shows a state in which the switch structure is viewed from a side surface. Fig. 4B shows a state in which the switch structure is viewed from the front surface.

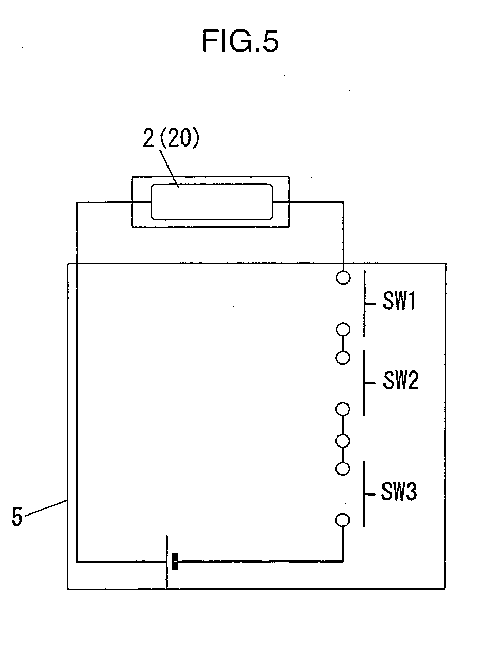

Fig. 5 is an explanatory drawing illustrating schematically the control circuit of the optical depilation apparatus.

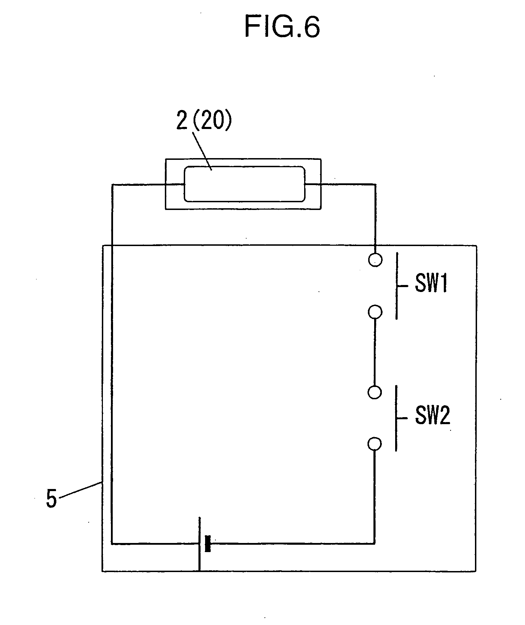

Fig. 6 is an explanatory drawing illustrating schematically a modification example of the control circuit.

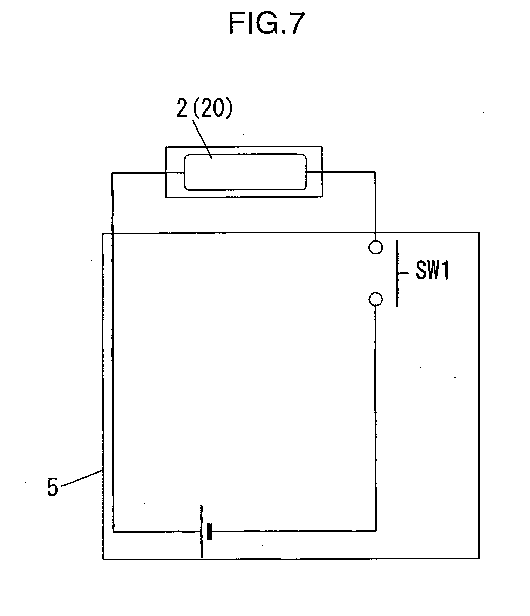

Fig. 7 is an explanatory drawing illustrating schematically another modification example of the control circuit.

### Best Mode for Carrying Out the Invention

The best mode for carrying out the invention will be described below in greater detail with reference to the appended drawings.

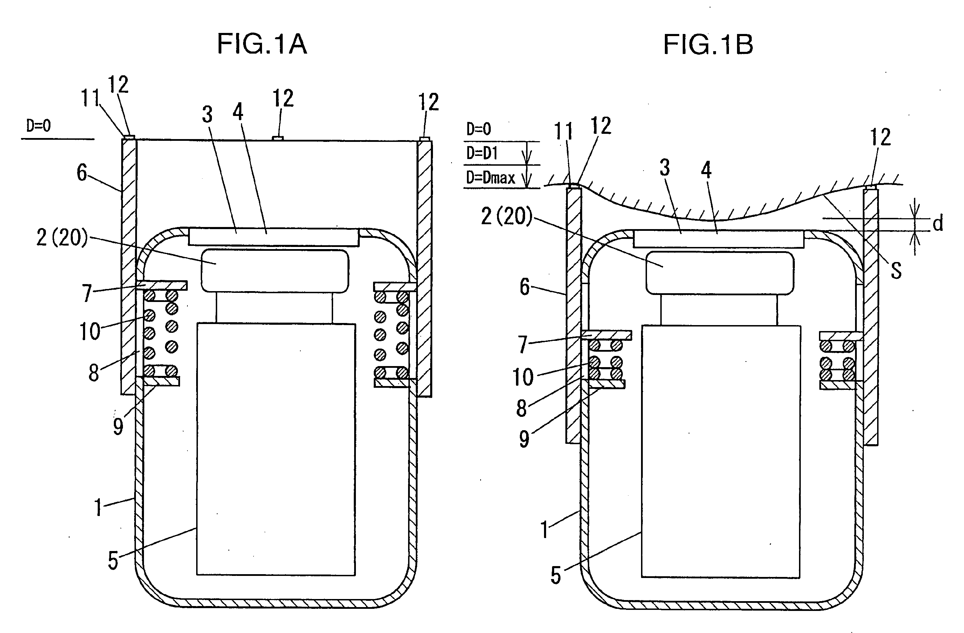

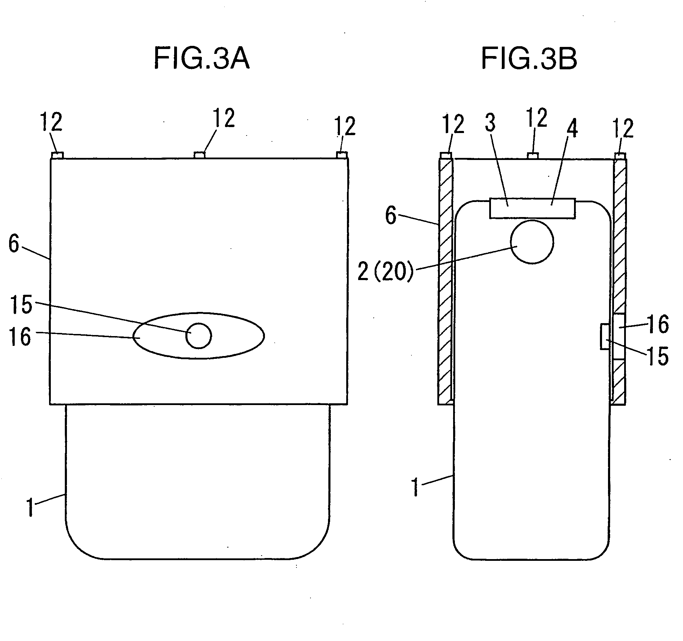

Figs. 1A, 1B to Figs. 3A, 3B illustrate schematically an embodiment of the optical depilation apparatus in accordance with the present invention. The optical depilation apparatus of the present embodiment is provided with a box-shaped apparatus body 1 that can be grasped by one hand. A flashlight 2 composed of a xenon light lamp is accommodated as a light source unit 20 in the apparatus body 1. A light irradiation port 3 is opened in a position close to the flashlight 2 at the distal end of the apparatus body 1. Pulsed flashes generated by the flashlight 2 are emitted through the light irradiation port 3 to the outside of the apparatus body 1. A transparent lamp cover 4 is fitted in the light irradiation port 3. The flashes emitted from the flashlight 2 are emitted through the lamp cover 4 to the outside of the apparatus body 1. The flashes are emitted upward in Figs. 1A and 1B. A control circuit 5 is incorporated in a state of connection to the flashlight 2 inside the apparatus body 1. The control circuit 5 raises a voltage applied from a power source to obtain a high voltage and applies the high voltage to the flashlight 2. The control circuit 5 also appropriately controls ON/OFF switching of light emission from the flashlight 2.

The optical depilation apparatus is provided with a light shielding part 6 that is movably supported by the apparatus body 1. The light shielding part 6 is formed as a pipe and made of a flexible elastic material such as rubber or elastomer. The light shielding part 6 is disposed so as to surround the light irradiation port 3 on the outer circumference of the apparatus body 1. Further, the light shielding part 6 is floatably attached to the apparatus 1. The "floatable state" as referred to herein means a state in which the light shielding part 6 is free to move back and forth along the light irradiation direction within a specific range, while the light shielding part 6 is being biased in the light irradiation direction. In the description below, the direction forward along the light irradiation direction will be referred to hereinbelow as "upward", and the direction rearward along the light irradiation direction will be referred to hereinbelow as "downward".

In the example shown in the figure, a linking convex portion 7, a receiving portion 9, and a spring member 10 are provided as means for floatably linking the light shielding part 6 to the apparatus body 1. Thus, in the present embodiment, the linking convex portion 7, receiving portion 9, and spring member 10 are provided as the support portions that support the light shielding part 6 movably with respect to the apparatus body 1. The linking convex portion 7 extends inward from the inner circumferential surface of the light shielding part 6, and the linking convex portion 7 is inserted into a linking port 8 opened at a side wall of the apparatus body 1. The linking port 8 has an opening sufficiently larger than the linking convex portion 7 in the light irradiation direction, and the linking convex portion 7 can freely move back and forth in the vertical direction in the linking port 8. The receiving portion 9 is provided in a position in the vicinity of the lower edge of the linking port 8 on the inner circumferential surface of the apparatus body 1. The spring member 10 is disposed between the linking convex portion 7 and receiving portion 9. Because the linking convex portion 7 is disposed above the receiving portion 9, the light shielding part 6 is biased in the light irradiation direction. In other words, the light shielding part 6 can move rearward and downward when pushed from above. The linking portion functioning as the linking means may also have a different configuration.

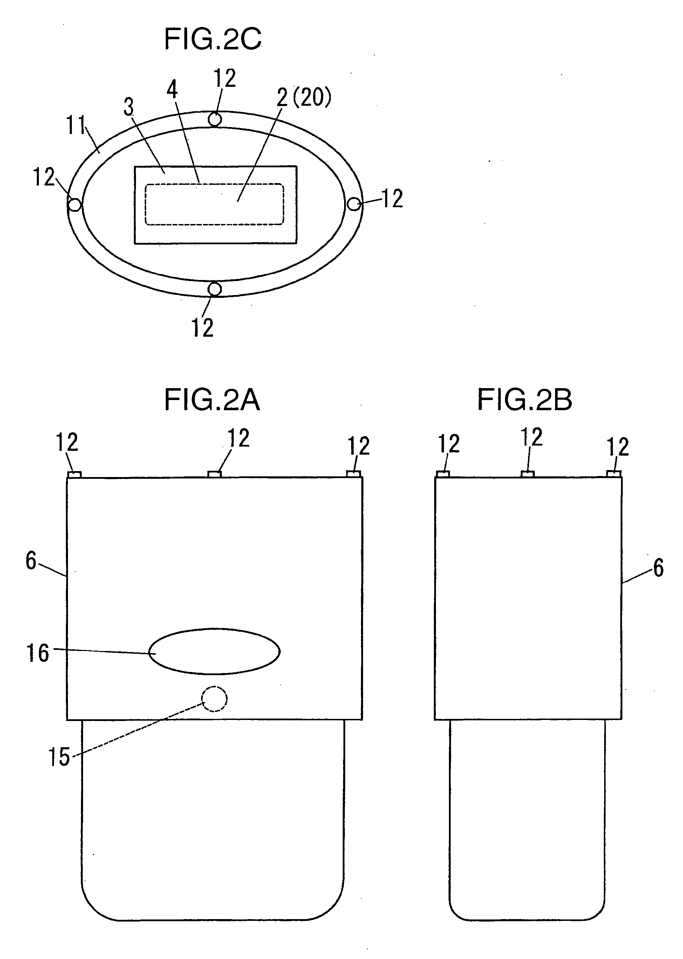

The distal end surface 11 of the tubular light shielding part 6 that is floatably mated with the outer circumferential surface of the apparatus body 1 is formed to have a ring-like shape in the plan view, as shown in Fig. 2C. The distal end surface 11 of the light shielding part 6 is a pressure surface that is to be directly pressed against the skin surface S of a human body. A plurality of contact sensors 12 are provided on the distal end surface 11. The contact sensors 12 are disposed at regular intervals in the circumferential direction. Each contact sensor 12 is electrically connected to the control circuit 5, and the control circuit 5 assumes that the skin surface S of the human body is in the intimate contact with the distal end portion of the light shielding part 6 and causes the flashlight 2 to emit light only when contact with the skin surface S is detected by all the contact sensors 12. In the example shown in the figure, the contact sensors 12 are disposed in four locations on the distal end surface 11, but this arrangement is not limiting. For example, only one contact sensor 12 may be provided on the distal end surface 11, or a plurality of contact sensors 12 may be arranged thereon. In a case where a plurality of contact sensors 12 are provided, they are not necessarily disposed at regular intervals.

Fig. 1A illustrates a state in which the light shielding part 6 is in the uppermost position, that is, a state in which a depression amount of the light shielding part 6 with respect to the apparatus body 1 is zero (D = 0). This is a state in which the light shielding part 6 is not pressed against the skin surface of the human body, and the light shielding part 6 in this case is in a natural state in which no pressing force is received thereby. Fig. 1B illustrates a state in which the light shielding part 6 is in the lowermost position, that is, a state in which the depression amount D is equal to the maximum depression amount Dmax. The vertical size or maximum depression amount Dmax of the light shielding part 6 is set such that the distal end surface 11 of the light shielding part 6 is positioned above the light irradiation port 3 of the apparatus body 1 in both states. Further, each size is set such that a gap d is ensured between the light irradiation port 3 and the skin surface S that bulges downward under the pressure of the distal end surface 11 even in a state in which the light shielding part 6 has been depressed to the maximum depression amount Dmax.

As shown in Fig. 4, a switch spring 13 is fixed to the inner circumferential surface of the light shielding part 6. The switch spring 13 comes into contact with a pair of connection terminals 14 located at the apparatus body 1 only in a case where the downward depression amount D of the light shielding part 6 with respect to the apparatus body 1 is equal to or greater than a predetermined amount D1(0 < D1 < Dmax). The switch spring 13 and connection terminals 14 are connected to the control circuit 5, and in a state in which the switch spring 13 and connection terminals 14 are not in contact with each other, the control circuit 5 is shut down.

As shown in Figs. 2 and 3, an irradiation switch 15 that enables the user to ON/OFF operate manually the emission of light from the flashlight 2 is provided at outer circumferential surface of the apparatus body 1. The irradiation switch 15 is provided in the center of the front side of the apparatus body 1. An operation port 16 is opened at the light shielding part 6 so as to expose the irradiation switch 15 when the light shielding part 6 is depressed downward by a predetermined amount or more. Thus, in a state in which the light shielding part 6 is not pushed down, the irradiation switch 15 is positioned outside the operation port 13, but in a state in which the light shielding part 6 is moved back to a degree such that the switching spring 13 and connection terminals 14 come into contact, the irradiation switch 15 is positioned in the operation port 16. The user can operate the irradiation switch 15 through the operation port 16. The control circuit 5 enables light emission from the flashlight 2 only in a case where the user continuously pushes the irradiation switch 15 exposed through the operation port 16 (or when the irradiation switch is fixed in the ON state). The irradiation switch 15 may not be disposed at the apparatus body 1 or, for example, in the intermediate portion or at the distal end of a cord extending from the apparatus body 1.

As shown schematically in Fig. 5, the control circuit 5 is provided with a switch SW1, a switch SW2, and a switch SW3 as the switches that can interrupt or connect power supply to the light source unit 20. These switches SW1 to SW3 are connected in series and constitute a triple switch circuit. The switch SW1 is constituted by the switch spring 13, connection terminals 14, or the like (see Fig. 4) so as to be switched ON synchronously when the depression amount D of the light shielding part 6 with respect to the apparatus body 1 is equal to or greater than a predetermined amount D1. The switch SW2 is electrically connected to each contact sensor 12 so as to be switched ON synchronously when all the contact sensors 12 provided at the distal end of the light shielding part 6 detect contact with the skin surface S. The switch SW3 is configured to be ON synchronously when the user pushes the irradiation switch 15 disposed at the apparatus body 1.

Therefore, the flashlight 2 emits pulsed flashes only when all the switches SW1, SW2, SW3 are ON. In other words, the flashlight 2 emits pulsed flashes only where the following conditions are satisfied: the depression amount D of the light shielding part 6 with respect to the apparatus body 1 is equal to or greater than the predetermined amount D1, the distal end of the light shielding part 6 is in intimate contact with the skin surface S and all the contact sensors 12 are ON, and the user manually operates the irradiation switch 15 of the apparatus body 1.

With the optical depilation apparatus of the present example having the above-described configuration, the user grasps the apparatus body 1 with one hand and brings the distal end surface 11 of the light shielding part 6 into contact with the skin surface S of the human body so as to position the skin surface S of the treatment object zone inside the region surrounded by the ringshaped distal end surface 11 of the light shielding part 6. Where the user then pushes the apparatus body 1 toward the skin surface S, the light shielding part 6 is pushed down, while the upward biasing force is increased, and the distal end surface 11 is brought into intimate contact with the skin surface S over the entire periphery of the distal end surface. In this state, even if the user somewhat moves the apparatus body 1, the light shielding part 6 that is in the floating state maintains the state of intimate contact with the skin surface S, while following this movement and moving up and down.

When the user then manually switches ON the irradiation switch 15 exposed from the operation port 16 of the light shielding part 6 in a state in which the depression amount D of the light shielding part 6 is equal to or greater than the predetermined amount D1 and every contact sensor 12 detects contact with the skin surface S, the flashlight 2 emits pulsed light. The light passes through the light irradiation port 3 and the skin surface S of the treatment object zone is irradiated by the light, thereby enabling the depilation. In this case, because the distal end surface 11 of the light shielding part 6 maintains the state of pressure contact with the skin surface S over the entire periphery of the distal end surface, the flash irradiated through the light irradiation port 3 is prevented from leaking to the outside, and the flash cannot enter the user's eyes. Further, because the light shielding part 6 is formed from a flexible elastic material such as rubber, the state of intimate contact is ensured more reliably.

Further, because the switch for ON/OFF switching the flashlight 2 is the triple switch, unless the user intentionally operates the irradiation switch 15 in a state in which the distal end surface 11 of the light shielding part 6 is brought into intimate contact with the skin surface S by a sufficient load over the entire periphery, the emission of light is disabled and safety is further increased.

For example, the switch in the control circuit 5 may be a double switch circuit in which the switch SW1 and switch SW2 are connected in series, as shown in Fig. 6, or a switch circuit composed only of the switch SW1, as shown in Fig. 7, rather than the triple switch circuit. In the case the double switch circuit shown in Fig. 6 is provided, the flashlight 2 emits pulsed light when the depression amount D of the light shielding part 6 with respect to the apparatus body 1 becomes equal to or greater than the predetermined amount D1, the distal end of the light shielding part 6 is brought into intimate contact with the skin surface S, and all the contact sensors 12 are ON. Further, in a case where the switch circuit shown in Fig. 7 is provided, the flashlight 2 emits pulsed light when the depression amount D of the light shielding part 6 with respect to the apparatus body 1 becomes equal to or greater than the predetermined amount D1.

Further, the light source unit 20 is not limited to the flashlight 2 composed of the xenon light lamp. For example, the light source unit may be the flashlight 2 of a different configuration. Alternatively, laser light may be emitted by using a laser diode or the like.

### (Summary of the Embodiment)

The above-described embodiment is summarized below.

(1) In the optical depilation apparatus of the present embodiment, the light shielding part can move in the light irradiation direction. Therefore, where the light source unit is caused to emit light in a state in which the light shielding part is pressed against the skin surface, the light that passes through the light irradiation port is blocked by the light shielding portion and prevented from leaking to the outside. Therefore, when the optical depilation apparatus is pressed against the skin surface and light is emitted, the light is reliably prevented from leaking from between the optical depilation apparatus and the skin surface and the depilation can be performed with high safety.

(2) It is preferred that the light shielding part be formed in a tubular shape that has a distal end surface that is to be pressed against a skin surface of a human body, and the support part support the light shielding part so that the distal end surface is positioned, in a natural state, downstream in the light irradiation direction with respect to the light irradiation port of the apparatus body. With such a configuration, the skin surface in a zone surrounded by the distal end surface can be encapsulated inside the tubular light shielding part. Therefore, light leak can be prevented even more reliably. The resultant effect is that safety can be further improved.

(3) It is preferred that the support part have a receiving portion provided at the apparatus body, a convex portion provided at the light shielding part, and a spring member disposed between the receiving part and the convex part. With such a configuration, the light shielding part is biased in the light irradiation direction by the elastic force of the spring member, and the light shielding part moves rearward as the distal end surface of the light shielding portion is pressed against the skin surface. Therefore, intimate contact of the light shielding part with the skin surface can be ensured even when the value of the force pressing the light shielding part against the skin surface changes.

(4) The optical depilation apparatus is preferably provided with a control circuit being capable of controlling light emission of the light source unit, and a switch that is connected to the control circuit and can interrupt power supply to the light source unit. With such a configuration, light emission of the light source unit can be controlled by operating the switch.

(5) In the optical depilation apparatus, it is preferred that the switch comprises a switch that is ON when a depression amount of the light shielding part with respect to the apparatus body is equal to or greater than a predetermined amount. With such a configuration, the light emitting unit does not emit light unless the light shielding part is pressed against the skin surface and brought into intimate contact with the skin surface by a pressing load that is equal to or higher than a certain level. Therefore, because the pressing load of the light shielding part can be ensured to be equal to or higher than a certain level, light leak can be prevented even more reliably.

(6) In the optical depilation apparatus, it is preferred that the switch comprises an irradiation switch that is connected to the control circuit and can be manually operated. With such a configuration, the light emitting unit does not emit light unless the user intentionally operates the irradiation switch. Therefore, the light emitting unit emits light only at a timing intended by the user. The resultant effect is that light leak can be prevented more reliably.

(7) It is preferred that an operation port be formed in the light shielding part and the irradiation switch be positioned so that the irradiation switch is in the operation port when the amount of depression of the light shielding part with respect to the apparatus body is equal to or greater than a predetermined amount. With such a configuration, the light emitting unit does not emit light even if the irradiation switch is operated in a state in which the light shielding part is not depressed to an amount equal to or greater than a predetermined amount. Therefore, light leak can be prevented even more reliably.

(8) It is preferred that the optical depilation apparatus further include a contact sensor disposed at a distal end portion of the light shielding part, and the switch comprises a switch that is ON when the contact sensor detects contact with a skin surface. With such a configuration, the light emitting unit does not emit light unless the light shielding part is in a state of intimate contact with the skin surface. Therefore, intimate contact of the light shielding part with the skin surface can be ensured. The resultant effect is that light leak can be prevented more reliably.

(9) It is preferred that the light shielding part be constituted by a flexible elastic body. With such a configuration, the light shielding part can be brought into gapless intimate contact with the skin surface. The resultant effect that light leak can be prevented even more reliably by bringing the flexible light shielding part into gapless intimate contact with the skin surface.

The above-described features can be appropriately combined without departing from the essence of the present invention.

## Claims

1. An optical depilation apparatus for performing depilation by irradiating skin with light, comprising:
an apparatus body having a light source unit and a light irradiation port and emitting light from the light source unit through the light irradiation port;
a light shielding part disposed so as to surround the light irradiation port of the apparatus body; and
a support part that supports the light shielding part so as to allow the light shielding part to move in a light irradiation direction with respect to the apparatus body.

2. The optical depilation apparatus according to claim 1, wherein
the light shielding part is formed in a tubular shape that has a distal end surface that is to be pressed against a skin surface of a human body; and
the support part supports the light shielding part so that the distal end surface is positioned, in a natural state, downstream in the light irradiation direction with respect to the light irradiation port of the apparatus body.

3. The optical depilation apparatus according to claim 1 or 2, wherein the support part has a receiving portion provided at the apparatus body, a convex portion provided at the light shielding part, and a spring member disposed between the receiving part and the convex part.

4. The optical depilation apparatus according to any one of claims 1 to 3, comprising:
a control circuit being capable of controlling light emission of the light source unit; and
a switch that is connected to the control circuit and can interrupt power supply to the light source unit.

5. The optical depilation apparatus according to claim 4, wherein the switch comprises a switch that is turned ON when an amount of depression of the light shielding part with respect to the apparatus body is equal to or greater than a predetermined amount.

6. The optical depilation apparatus according to claim 4, wherein the switch comprises an irradiation switch that is connected to the control circuit and can be manually operated.

7. The optical depilation apparatus according to claim 6, wherein
an operation port is formed in the light shielding part, and
the irradiation switch is positioned so that the irradiation switch is in the operation port when the amount of depression of the light shielding part with respect to the apparatus body is equal to or greater than a predetermined amount.

8. The optical depilation apparatus according to claim 4, further comprising a contact sensor disposed at a distal end portion of the light shielding part, wherein
the switch comprises a switch that is turned ON when the contact sensor detects contact with a skin surface.

9. The optical depilation apparatus according to any one of claims 1 to 8, wherein the light shielding part is constituted by a flexible elastic body.
